# EUROPÄISCHE PATENTSCHRIFT

(11) **EP 0 851 259 B1**
(45) Veröffentlichungstag und Bekanntmachung des Hinweises auf die Patenterteilung: **07.05.2003**
(21) Anmeldenummer: 97117791.0
(22) Anmeldetag: 14.10.1997
(51) Int. Cl.: G02B 7/00

(54) **Aufhängung für ein Operationsmikroskop**
Suspension system for surgical microscope
Suspension pour un microscope chirugical

(30) Priorität: 24.12.1996 DE 19654302
(43) Veröffentlichungstag der Anmeldung: 01.07.1998
(73) Patentinhaber: Möller-Wedel GmbH, 22880 Wedel (DE)
(72) Erfinder: Duis, Wilhelm, 25491 Hetlingen (DE)
(74) Vertreter: Glawe, Delfs, Moll & Partner

(56) Entgegenhaltungen:
- EP-A- 0 048 404
- WO-A-91/00472
- US-A- 4 447 031
- US-A- 5 288 043

## Beschreibung

Die Erfindung betrifft eine Aufhängung für ein Operationsmikroskop mit mindestens einem vertikal verstellbaren, als Parallelogrammarm ausgebildeten Tragarm mit einem oberen und einem gleichlangen unteren Arm, deren eine Enden schwenkbar an einem ersten Endstück an zwei ersten Schwenkpunkten und deren andere Enden schwenkbar an einem zweiten Endstück an zwei zweiten Schwenkpunkten befestigt sind, deren Abstand gleich dem der ersten Schwenkpunkte ist, wobei Federmittel zum Gewichtsausgleich vorgesehen sind und das Mikroskop mit dem zweiten Endstück verbunden ist.

Operationsmikroskope werden in bestimmten chirurgischen Fachgebieten benötigt, wenn immer kleine biologische Strukturen operativ behandelt werden müssen. In bestimmten Fachgebieten wie z. B. in der Neurochirurgie muß dabei das Operationsmikroskop an einer Aufhängevorrichtung angebracht sein, die eine freie Bewegung des Mikroskops in den drei Raumachsen sowie den Drehachsen des Mikroskops mit geringsten Kräften ermöglicht. Die Minimierung des Kraftaufwands bei der Bewegung ist dabei von großer Bedeutung, da der Chirurg zum einen die während der Operation benötigte Feinfühligkeit nicht durch Kraftanstrengungen für die Mikroskopbewegung verlieren darf, zum weiteren, da er, um beidhändig operieren zu können, das Mikroskop häufig mit einem Mundschalter bewegen muß. Außer in der Neurochirurgie werden ähnliche Forderungen auch in anderen Fachgebieten gestellt.

Gefordert ist daher eine Aufhängung für Operationsmikroskope, die einen optimalen Gewichtsausgleich bei gleichzeitig minimalen Reibungskräften in allen Bewegungsrichtungen und Raumlagen ermöglicht.

Es gibt auf dem Markt Mikroskopaufhängungen nach dem Balance-Prinzip, bei deren der Gewichtsausgleich durch Gegengewichte erzeugt wird. Systeme dieser Art haben jedoch Nachteile in der Praxis. Zum einen ist die Austarierung des Schwebezustands sehr aufwendig und erfordert großes Geschick von der Operationsschwester. Weiterhin nachteilig ist die Neigung eines solchen Systems zum Nachschwingen nach einer Bewegung, da, physikalisch gesehen, zwei große Massen durch ein quasifederndes Element verbunden sind. Weiterhin muß der Chirurg bei der Bewegung des Mikroskops eine große Massenträgheit überwinden, da außer dem Mikroskop jeweils noch Gegengewichte bewegt werden müssen.

Es gibt auch Mikroskopaufhängungen nach dem Balance-Prinzip, die eine automatische Austarierung des Schwebezustands ermöglichen. Jedoch bleiben auch die anderen Nachteile dieses Bauprinzips bestehen. Außerdem werden diese Geräte durch die zusätzlichen Maßnahmen unverhältnismäßig teuer.

Mikroskopsaufhängungen anderer Art arbeiten nach einem Doppelarmprinzip. Dabei wird die horizontale Bewegung durch Drehbewegungen zweier Tragarme ermöglicht, wobei einer der Tragarme als Parallelogrammarm mit Federvorspannung ausgebildet ist, der auch eine Vertikalbewegung zuläßt. Während die Horizontalbewegungen sehr reibungsarm ausgeführt werden können, ist der Gewichtsausgleich durch Federkräfte nur unvollkommen zu realisieren. Meistens kommen Gasfedern zum Einsatz. Dabei ist jedoch nachteilig, daß Gasfedern grundsätzlich eine Reibung durch die Gasdichtung aufweisen. Mechanische Federn, insbesondere Spiral-Zugfedern, können dagegen wegen der Abhängigkeit der Kraft von der Ausdehnung nur unvollkommen zur Gewichtskompensation eingesetzt werden.

Dies liegt daran, daß die Federkraft als Funktion der Vertikalstellung des Mikroskops trigonometrischen Funktionen folgt, die nur in sehr kleinen Bereichen durch eine Gerade angenähert werden können.

Die Aufgabe der Erfindung besteht in der Schaffung einer Aufhängung für ein Operationsmikroskop, bei der durch einen verbesserten Gewichtsausgleich die oben genannten Nachteile vermieden werden, insbesondere eine über einen größeren Bereich im wesentlichen lineare bzw. konstante Gewichtskompensation erzielt wird.

Die erfindungsgemäße Lösung besteht darin, daß mindestens zwei Federmittel vorgesehen sind, wobei die ersten Federmittel zwischen dem oberen und unteren Arm oder zwischen einem Schwenkpunkt eines Arms und dem anderen Arm und die zweiten Federmittel zwischen einem Endstück und einem der Arme verbunden sind.

Die Mikroskopaufhängung verwendet den bereits erwähnten Paralellogrammarm mit Gewichtsausgleich durch Federmittel, wobei jeweils zwei oder mehr Federmittel vorgesehen sind, von denen eines eine Kraftwirkung zwischen den Armen des Paralellogrammarms und ein weiteres eine Kraftwirkung zwischen einem Arm und einem Endstück des Paralellogrammarms ausübt. Konventionelle Paralellogrammarmaufhängungen weisen dagegen pro Paralellogrammarm nur ein solches Federmittel auf (siehe EP 0 048 404 oder WO 91/00 472). Durch die Kombination von zwei oder mehr Federmitteln, die wie in den Ansprüchen angegeben angeordnet sind, kann ein Gewichtsausgleich über einen größeren Bereich erzielt werden. Dabei soll in der genannten Beschreibung eine in den Ansprüchen, wie dies unten noch näher ausgeführt werden wird, nicht nur eine Einzelfeder, sondern auch Sätze von Einzelfedern verstanden werden, die parallel zueinander, im wesentlichen an den gleichen Stellung und gleichwirkend angeordnet sind.

Die Erfindung wird im folgenden anhand von vorteilhaften Ausführungsformen unter Bezugnahme auf die beigefügten Zeichnungen beschrieben. Es zeigen:
- Fig. 1: in schematischer Ansicht eine erste Ausführungsform mit Schraubenfedern; und
- Fig. 2: in ähnlicher Darstellung wie in Fig. 1 eine zweite Ausführungsform mit Druckfedern.

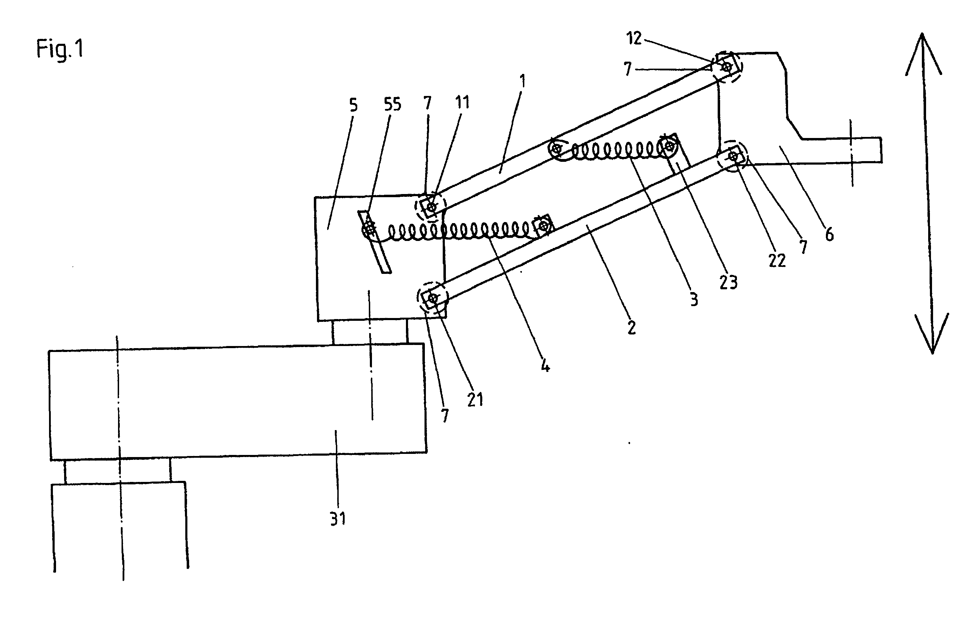

Es wird zunächst auf Fig. 1 bezug genommen. Der Tragarm besteht aus zwei Armen 1 und 2, die über Schwenkpunkte 11 und 21 in Form von Scharnieren, Schwenkzapfen oder dergleichen mit einem ersten oder hinteren Endstück 5 verbunden sind, das drehbar an einem zweiten Tragarm 31 angebracht ist. An ihren gegenüberliegenden Enden sind die Arme 1 und 2 über Schwenkpunkte 12, 22 mit dem vorderen oder zweiten Endstück 6 verbunden, das eine Aufnahmevorrichtung für das Mikroskop enthält. Der Abstand der Schwenkpunkte 11 und 21 ist dabei gleich dem Abstand der Schwenkpunkte 12 und 22, und die Arme 1 und 2 sind gleichlang, so daß sich ein Paralellogrammtragarm ergibt. An den Armen 1 und 2 sind Federmittel 3 in Form von einer oder mehreren Zugfedern angebracht, deren Kraftwirkung dem Gewicht des zweiten Endstücks 6 entgegenwirkt. In Fig. 1 ist gezeigt, daß die Federmittel am Träger 1 direkt und am Träger 2 über ein Ansatzstück 23 angebracht sind. Auf dieses Ansatzstück 23 kann bei anderen Ausführungsformen verzichtet werden, oder es kann auch für den Arm 1 ein entsprechendes Ansatzstück verwendet werden.

Zusätzlich zu den ersten Federmitteln 3 sind zweite Zugfedermittel 4 vorgesehen, die zwischen dem zweiten Arm 2 und einem Punkt 55 am ersten Endstück verbunden sind. Dieser Befestigungspunkt 55 für die zweiten Federmittel 4 kann dabei z. B. mit einer Spindel noch verstellt werden, um so den Gewichtsausgleich zu optimieren.

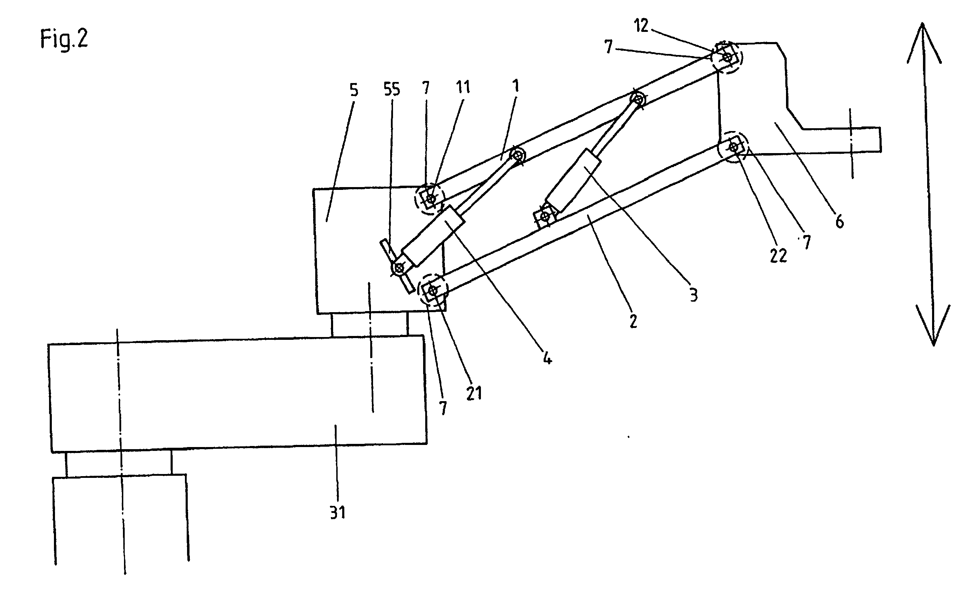

Bei der in Fig. 1 dargestellten Ausführungsform sind Zugfedern verwendet. Bei der in Fig. 2 dargestellten Ausführungsform sind Druckfedern verwendet. Der einzige wesentliche Unterschied zu Zugfedern besteht darin, daß die Kraftansatzpunkte am Arm 1, am Arm 2 und am Endstück 5 oder 6 anders angeordnet sind. Selbstverständlich ist auch eine Kombination von Zugfedermitteln und Druckfedermitteln möglich.

Zweckmäßigerweise sind als Federmittel jeweils nicht nur eine Feder, sondern zwei oder noch mehr Federn vorgesehen. Dadurch kann einerseits eine einseitige Belastung der Arme 1, 2 verhindert werden. Andererseits wird der Gewichtsausgleich nicht völlig funktionslos, wenn eine Feder brechen sollte. Insbesondere könnten auch pro Federmittel mehr als zwei Federn vorgesehen sein. Wenn mechanische Zugfedern verwendet werden, so ist dadurch im Gegensatz zu Gasdruckfedern eine reibungsfreie Bewegung und eine gewisse Sicherheit gegen Bruch einer Feder erreicht. Zweckmäßigerweise wird der Tragarm auch mit einer elektrisch betätigten Bremse ausgestattet, die ihn auf einer vom Benutzer vorgegebenen Höhe hält. Die Bremse muß jedoch dabei so weich eingestellt sein, daß bei Stromausfall notfalls eine Höhenverstellung des Mikroskops gegen die ungelöste Bremse möglich ist. Das Ungleichgewicht bei Ausfall einer Feder darf deshalb die Haltekraft der Bremse nicht übersteigen. Durch eine größere Anzahl von Federn kann die Gesamtkraft so verteilt werden, daß bei Federbruch das Ungleichgewicht nicht allzu groß wird. Die Bremsen sind schematisch bei 7 gestrichelt angedeutet.

Praktische Versuche haben gezeigt, daß über einen weiten Winkelbereich des Tragarms ein Gewichtsausgleich zu erreichen ist, der bei einem Gesamtgewicht von z. B. 12 Kg eine Restkraft von weniger als 0,5 N ermöglicht. Eine derart kleine Restkraft erlaubt den Einsatz eines Mundschalters und ist im praktischen Betrieb mit der Hand nahezu nicht mehr wahrnehmbar.

Die Erfindung wurde anhand einer vorteilhaften Ausführungsform mit zwei Federmitteln 3, 4 beschrieben. Im Rahmen der Erfindung ist aber nicht nur eine andere Anordnung dieser beiden Federmittel möglich, wie dies zum Teil auch erwähnt wurde. Es ist vielmehr auch möglich, noch mehr Federmittel einzusetzen, in dem man z. B. Tragarme nicht nur mit einem Endstück, sondern durch ein weiteres Federmittel auch mit dem zweiten Endstück verbindet.

## Patentansprüche

1. Aufhängung für ein Operationsmikroskop mit mindestens einem vertikal verstellbaren, als Paralellogrammarm ausgebildeten Tragarm mit einem oberen und einem gleichlangen unteren Arm, deren eine Enden schwenkbar an einem ersten Endstück an zwei ersten Schwenkpunkten und deren andere Enden schwenkbar an einem zweiten Endstück an zwei zweiten Schwenkpunkten befestigt sind, deren Abstand gleich dem der ersten Schwenkpunkte ist, wobei Federmittel zum Gewichtsausgleich vorgesehen sind, und das Mikroskop mit dem zweiten Endstück verbunden ist, **dadurch gekennzeichnet, daß** mindestens zwei Federmittel (3, 4) vorgesehen sind, wobei die ersten Federmittel (3) zwischen dem oberen und unteren Arm (1, 2) oder zwischen einem Schwenkpunkt (11, 12; 21, 22) eines Arms (1; 2) und dem anderen Arm (2; 1) und die zweiten Federmittel (4) zwischen einem Endstück (5, 6) und einem der Arme (1, 2) verbunden sind.

2. Aufhängung nach Anspruch 1, **dadurch gekennzeichnet, daß** die zweiten Federmittel (4) mit dem Endstück (5, 6) an einer von Schwenkpunkten verschiedenen Stelle (55) verbunden sind.

3. Aufhängung nach Anspruch 1 oder 2, **dadurch gekennzeichnet, daß** die zweiten Federmittel (4) mit dem ersten Endstück (5) verbunden sind.

4. Aufhängung nach einem der Ansprüche 1 bis 3, **dadurch gekennzeichnet, daß** der Befestigungspunkt (55) der zweiten Federmittel (4) am Endstück (5) verstellbar ausgebildet ist.

5. Aufhängung nach einem der Ansprüche 1 bis 4, **dadurch gekennzeichnet, daß** die Federmittel (3, 4) wenigstens zum Teil mit dem jeweiligen Arm (1, 2) über ein Ansatzstück (23) desselben verbunden sind.

6. Aufhängung nach einem der Ansprüche 1 bis 5, **dadurch gekennzeichnet, daß** jedes Federmittel (3, 4) aus mindestens zwei Einzelfedern besteht.

7. Aufhängung nach einem der Ansprüche 1 bis 6, **dadurch gekennzeichnet, daß** die Federmittel (3, 4) Zugfedern aufweisen.

8. Aufhängung nach Anspruch 7, **dadurch gekennzeichnet, daß** die Zugfedern Schraubenfedern sind.

9. Aufhängung nach einem der Ansprüche 1 bis 6, **dadurch gekennzeichnet, daß** die Federmittel (3, 4) Druckfedern aufweisen.

10. Aufhängung nach einem der Ansprüche 1 bis 9, **dadurch gekennzeichnet, daß** sie elektrisch betätigte Bremsen (7) für die Bewegung des Tragarms oder der Tragarme aufweist.

## Claims

1. A suspension for a surgical microscope having at least one vertically adjustable support arm, which is constructed as a parallelogram arm, has an upper arm and a lower arm of the same length, the first ends of which are fastened pivotably to a first end piece at two first pivot points and the other ends of which are fastened pivotably to a second end piece at two second pivot points whose spacing is equal to that of the first pivot points, spring means being provided for balancing, and a microscope being connected to a second end piece, wherein at least two spring means (3, 4) are provided, the first spring means (3) being connected between the upper and lower arms (1, 2) or between a pivot point (11, 12; 21, 22) of one arm (1; 2) and the other arm (2; 1), and the second spring means (4) being connected between one end piece (5, 6) and one of the arms (1, 2).

2. The suspension as claimed in claim 1, wherein the second spring means (4) are connected to the end piece (5, 6) at a point (55) different from pivot points.

3. The suspension as claimed in claim 1 or 2, wherein the second spring means (4) are connected to the first end piece (5).

4. The suspension as claimed in one of claims 1 to 3, wherein the fastening point (55) of the second spring means (4) is constructed adjustably on the end piece (5).

5. The suspension as claimed in one of claims 1 to 4, wherein the spring means (3, 4) are at least partially connected to the respective arm (1, 2) via an attachment piece (23) thereof.

6. The suspension as claimed in one of claims 1 to 5, wherein each spring means (3, 4) comprises at least two individual springs.

7. The suspension as claimed in one of claims 1 to 6, wherein the spring means (3, 4) have tension springs.

8. The suspension as claimed in claim 7, wherein the tension springs are helical springs.

9. The suspension as claimed in one of claims 1 to 6, wherein the spring means (3, 4) have compression springs.

10. The suspension as claimed in one of claims 1 to 9, wherein it has electrically actuated brakes (7) for the movement of the support arm or of the support arms.

## Revendications

1. Suspension pour microscope chirurgical comprenant au moins un bras porteur réglable verticalement, en forme de parallélogramme, comprenant un bras supérieur et un bras inférieur de la même longueur, dont l'une des extrémités est fixée de manière orientable à une première pièce d'extrémité à deux premiers points de pivotement et dont l'autre des extrémités est fixée de manière orientable à une deuxième pièce d'extrémité à deux deuxièmes points de pivotement, dont l'écart est égal à celui des premiers points de pivotement, des moyens formant ressort étant prévus pour équilibrer le poids, et le microscope étant relié à la deuxième pièce d'extrémité, **caractérisée en ce qu'**au moins deux moyens formant ressort (3, 4) sont prévus, les premiers moyens formant ressort (3) étant reliés entre le bras supérieur et inférieur (1, 2) ou entre un point de pivotement (11, 12 ; 21, 22) d'un bras (1 ; 2) et de l'autre bras (2 ; 1) et les deuxièmes moyens formant ressort (4) entre une pièce d'extrémité (5, 6) et l'un des bras (1, 2).

2. Suspension selon la revendication 1, **caractérisée en ce que** les deuxièmes moyens formant ressort (4) sont reliés à la pièce d'extrémité (5, 6) à un endroit (55) différent des points de pivotement.

3. Suspension selon la revendication 1 ou 2, **caractérisée en ce que** les deuxièmes moyens formant ressort (4) sont reliés à la première pièce d'extrémité (5).

4. Suspension selon l'une des revendications 1 à 3, **caractérisée en ce que** le point de fixation (55) des deuxièmes moyens formant ressort (4) est conçu de manière réglable sur la pièce d'extrémité (5).

5. Suspension selon l'une des revendications 1 à 4, **caractérisée en ce que** les moyens formant ressort (3, 4) sont reliés au moins en partie au bras respectif (1, 2) au-dessus d'un embout (23) de celui-ci.

6. Suspension selon l'une des revendications 1 à 5, **caractérisée en ce que** chaque moyen formant ressort (3, 4) se compose d'au moins deux ressorts individuels.

7. Suspension selon l'une des revendications 1 à 6, **caractérisée en ce que** les moyens formant ressort (3, 4) présentent des ressorts de traction.

8. Suspension selon la revendication 7, **caractérisée en ce que** les ressorts de traction sont des ressorts à boudin.

9. Suspension selon l'une des revendications 1 à 6, **caractérisée en ce que** les moyens formant ressort (3, 4) présentent des ressorts de pression.

10. Suspension selon l'une des revendications 1 à 9, **caractérisée en ce qu'**elle présente des freins (7) actionnés électriquement pour le mouvement du bras porteur ou des bras porteurs.
